# DEMANDE DE BREVET EUROPEEN

(11) **EP 1 318 400 A1**
(43) Date de publication de la demande: **11.06.2003**
(21) Numéro de dépôt: 01870273.8
(22) Date de dépôt: 06.12.2001
(51) Int. Cl.: G01N 33/543, C12Q 1/68

(54) **Procédé pour effectuer un diagnostic in vitro au moyen de mécanismes de régulation génétique et trousse de diagnostic correspondante**

(71) Demandeur: Unisensor S.A., 4000 Liege (BE)
(72) Inventeur: Granier, Benoît, 4120 Rotheux-Rimiere (BE); Lepage, Sophie, 4540 Amay (BE)
(74) Mandataire: Van Malderen, Michel

(57) **Abrégé**

La présente invention se rapporte à une trousse de détection et/ou de quantification d'un ligand (C), présent dans un échantillon d'analyse, par un récepteur (A), caractérisée en ce qu'elle comprend les réactifs suivants :
- une séquence nucléotidique (B) simple brin ou double brin, et
- ledit récepteur (A) constitué d'une entité protéique monomérique ou multimérique comprenant au moins un premier site de reconnaissance spécifique pour ledit ligand (C) et un deuxième site de reconnaissance spécifique pour ladite séquence nucléotidique (B),
ledit ligand (C) étant capable de moduler positivement ou négativement la fixation du récepteur (A) par son site de reconnaissance spécifique sur la séquence nucléotidique (B).

## Description

### Objet de l'invention

La présente invention concerne un procédé utilisant totalement ou partiellement *in vitro* des éléments connus appartenant aux mécanismes de régulation de l'expression des gènes, qui aurait principalement comme champ d'application la reconnaissance et le dosage de molécules spécifiques telles que des antibiotiques.

L'invention concerne également une trousse de diagnostic mettant en oeuvre le procédé.

### Arrière-plan scientifique

Les systèmes de régulation génétique naturels sont définis comme des mécanismes pouvant moduler l'expression des gènes suite à la présence ou à l'absence de certaines molécules spécifiques dans l'environnement cellulaire.

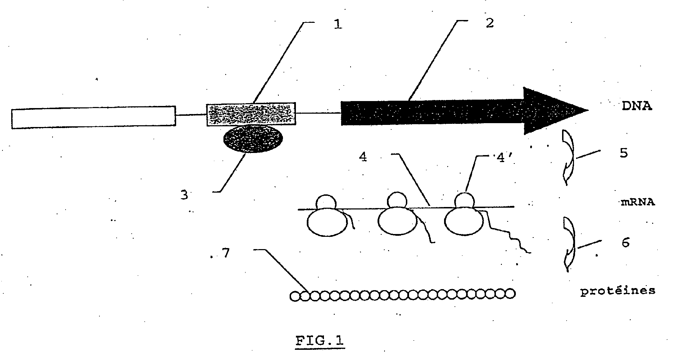

L'expression des gènes peut être contrôlée au cours des étapes de transcription, d'élongation et de traduction des acides nucléiques (figure 1).

On distingue le contrôle négatif et le contrôle positif de la transcription. Dans le premier cas, un récepteur protéique, appelé répresseur, empêche le gène d'être exprimé en se fixant sur l'opérateur de celui-ci et empêche ainsi la RNA-polymérase d'initier la transcription sur le promoteur. Ce système est très fréquent chez les bactéries (TetR, VarR, LacI). Dans le deuxième cas, un activateur ou facteur de transcription se fixant sur le DNA est nécessaire pour initier la transcription. Ce système est fréquent chez les eucaryotes (par ex. récepteurs hormonaux) mais est également présent chez les bactéries, comme c'est le cas de l'activateur AmpR régulant la transcription de la β-lactamase AmpC chez les bactéries à Gram négatif comme *Citrobacter freundei* (Jacobs *et al.,* Cell, vol.88 (1997), pp. 823-832) ou de PurR (Schumacher *et al.,* Cell, vol.83 (1995), pp. 147-155). De manière analogue, la traduction peut également être régulée par la fixation d'un répresseur sur le mRNA qui empêche le ribosome d'initier la traduction en protéines. Chez les eucaryotes, l'élongation du mRNA peut en plus être régulée lors des étapes de modification, "splicing", transport ou stabilité.

Les régulateurs sont des entités protéiques qui peuvent se trouver dans 2 états : soit dans un état activé (RTA) où ils peuvent, en se fixant sur le DNA ou le mRNA, réguler de manière positive (+) ou négative (-) l'expression des gènes, et donc la synthèse des protéines, soit dans un état inactivé (RTI) ou ils ne peuvent plus se lier à une séquence nucléotidique particulière. L'activation est généralement due à une transition allostérique du répresseur suite à la présence ou l'absence d'un ligand de haute affinité sur un site spécifique du régulateur. C'est le cas des régulateurs bactériens appartenant à la famille LacI (Weickert and Adhya, J. Biol. Chem., vol.267 (1992), pp. 15869-74) ou des récepteurs hormonaux chez les eucaryotes. D'autres phénomènes biochimiques comme l'oxydation, la phosphorylation ou la glycosylation peuvent également "allumer" ces régulateurs.

Dans ce cas du mécanisme lié à la fixation d'un ligand, les régulateurs possèdent deux sites de fixation :
- d'une part un site de fixation au DNA souvent caractérisé par un motif exposé hélice α - tournant β - hélice α (HTH), caractéristique des protéines se liant au DNA ;
- d'autre part un site de fixation au ligand.

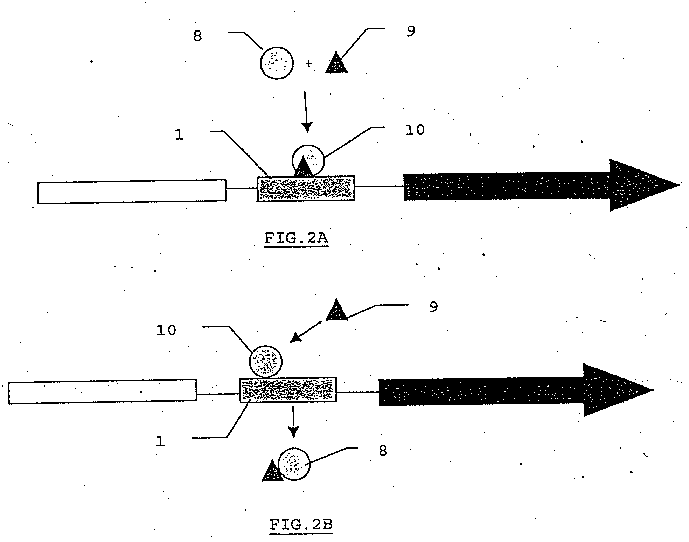

La présence du ligand , appelé effecteur, sur le régulateur induit généralement une modification de conformation de celui-ci qui va permettre (ou au contraire empêcher) la fixation de ce régulateur sur une séquence nucléotidique déterminée. Ces effecteurs agissent donc comme inducteurs ou co-répresseurs. Ces cas de figures les plus fréquents sont illustrés aux figures 2A et 2B.

C'est le cas de la purine, co-répresseur nécessaire à la fixation du répresseur dimérique PurR, appartenant à la famille des régulateurs transcriptionnels bactériens LacI, sur l'opérateur PurF (Schumacher *et al*., Cell, vol.83 (1995), pp. 147-155 ; Schumacher *et al*., Science, vol.266 (1994), pp. 763-770) ou du L-tryptophane, co-répresseur du dimère Trp (Otwinowski *et al.,* Nature, vol.335 (1988), pp. 321-329).

La plupart des autres régulateurs bactériens de la famille de LacI, à l'exception de PurR, répriment un chemin catabolique et non biosynthétique, l'affinité des régulateurs pour l'opérateur étant supérieure en l'absence de ligand sur le répresseur. Ainsi les régulateurs TetR et VarR sont des dimères appartenant également à la famille LacI qui interviennent dans les mécanismes de résistance bactérienne aux antibiotiques tétracyclines et virginiamycines respectivement. Ces répresseurs, présentant d'ailleurs une forte homologie de séquence, peuvent se fixer sur les opérateurs "tet" et "var" uniquement en absence de leurs ligands respectifs, tétracycline ou dérivés et virginiamycine S considérés comme des effecteurs inducteurs (Hillen *et al*., Ann. Rev. Microbiol., vol.48 (1994), p. 345 ; Namwat *et al.,* J. Bac., vol.183 (Mar. 2001), pp. 2025-2031).

En présence d'un inducteur fixé, les régulateurs "lâchent" l'opérateur et la répression est immédiatement levée. On observe une synthèse de TetA et de VarS, transporteurs membranaires catalysant le transport des antibiotiques hors de la cellule. Ce système nécessite une sensibilité très contrôlée puisqu'il régule la toxicité du transporteur qui va à la fois empêcher l'antibiotique d'atteindre sa cible (le ribosome) mais peut également relarguer d'autres ions non spécifiques hors de la cellule, ce qui est également létal pour la cellule. TetR doit donc maintenir une forte répression sur TetA jusqu'à ce qu'un faible niveau de tétracycline (Tc) soit présent.

Pour ce qui est de TetR, la structure tridimensionnelle est connue en présence de Tc ainsi qu'en présence de l'opérateur (Hinrichs *et al.,* Science, vol.264 (1994), pp. 418-420 ; Kisker *et al*., J. Mol. Biol., vol.247 (1995), pp. 260-280 ; Orth *et al.,* Nature Structural Biol., vol.7 (2000), pp. 215-219). Elle permet de mieux comprendre la machinerie moléculaire. Une orientation précise d'une chaîne latérale des acides aminés terminaux est requise pour permettre un contact de haute affinité entre la région opératrice du DNA et le régulateur. Quand la tétracycline se fixe, la distance centre à centre entre les deux motifs HTH du dimère de TetR augmente de 5 Å et cette légère modification de structure suffit à interrompre le contact de haute affinité avec l'opérateur. Les études *in vitro* montrent que l'affinité de TetR pour l'opérateur en absence de Tc est de 10¹² à 10¹³ M⁻¹ et chute d'un facteur ∼10⁹ en présence de Tc. TetR constitue le système de régulation transcriptionnel inductible le plus efficace connu à ce jour (Orth *et al*., Nature Structural Biol., vol.7 (2000), pp. 215-219).

Par ailleurs, il faut noter qu'en fonction des homologies de séquence, six classes (A, B, C, D, E et G) de TetR sont connues chez les bactéries à Gram négatif. Les gènes qui codent pour ces récepteurs sont plasmidiens ou portés par des transposons et ils sont tous inductibles à des concentrations nanomolaires en tétracycline. Les protéines présentent 29% d'acides aminés identiques entre les différentes classes ce qui suppose une structure 3D probablement similaire (Hillen *et al.,* Annu. Rev. Microbiol., vol.48 (1994), pp. 345-332 ; Klock *et al*., J. Bac., vol.161 (1985), pp. 326-332).

Pour ce qui est de VarR, les paramètres d'affinité en présence et en absence d'inducteur ne sont pas encore connus mais il est probable qu'ils seraient du même ordre de grandeur. L'affinité de l'antibiotique pour sa cible, le ribosome 50S, étant semblable, un haut delta entre la répression et l'induction du transporteur membranaire est également vital pour la cellule.

D'autres répresseurs moins vitaux comme LacI ne montrent qu'une perte d'activité de 10⁵ en présence de l'inducteur (Matthews *et al*., Nature Structure Biol., vol.7 (2000), pp. 184-187).

Là encore, il faut noter qu'il existe des exceptions puisque AmpR fixé sur l'opérateur peut activer la transcription *in vitro* en absence de ligand ou en présence du ligand anhydro-Mur Nac tripeptide. Par contre, en présence d'un autre ligand d'AmpR, l'UDP-Mur-Nac pentapeptide, la transcription est inhibée (Jacobs *et al*., Cell, vol.88 (1997), pp. 823-832). AraC se fixe sur le DNA en présence et en absence de ligand (L-arabinose) mais la transcription n'est initiée qu'en présence de ligand suite à un DNA "looping" (Soisson *et al*., Science, vol.276 (1997), pp. 421-425).

D'autres répresseurs encore, comme MetJ, se fixent sur l'opérateur uniquement en présence du co-répresseur chargé positivement (s-adenosylméthionine) qui induit non pas un changement de conformation mais un effet électrostatique au niveau du régulateur ce qui augmente son affinité de plus de 1000 fois pour le DNA (Phillips and Phillips, Structure, vol.2 (1994), pp. 309-316).

### Etat de la technique

A ce jour, aucune invention, c'est-à-dire aucune coordination de moyens techniques, n'a mis en évidence la possibilité ou la faisabilité de l'exploitation de l'ensemble des molécules, ni en particulier uniquement certaines molécules, impliquées dans les mécanismes de régulation génétique, pour le développement d'outils de diagnostic *in vitro* dans le but de détecter la présence et/ou de quantifier la teneur de certaines substances éventuellement présentes dans un échantillon à analyser.

La présente invention ne prétend pas faire appel à un quelconque développement cellulaire en culture pour fonctionner et ne peut concerner les applications dans lesquelles c'est le mécanisme de régulation génétique lui-même qui est cloné et exploité *in vivo* dans une souche bactérienne ou une lignée cellulaire. Dans ce dernier cas bien particulier, le développement cellulaire ou la croissance bactérienne sur milieu nutritif est en effet incontournable pour permettre *in vivo* l'expression du mécanisme de régulation génétique. Certaines applications de ce type renseignent la possibilité, au départ de cellules recombinantes génétiquement, de réaliser des mesures et de doser d'éventuelles substances présentes dans un échantillon. C'est notamment le cas pour le dosage des dioxines connu sous le nom de Calux® (Chemical Activated LUciferase gene eXpression) décrit dans le brevet américain n° US-A-5,854,010 (Bioassay for detecting 2,3,7,8-tetrachlorodibenzo-paradioxin and TCDD-like compounds and novel recombinant cell line useful therefor). Ce kit de diagnostic fonctionne pour le dosage des dioxines et leurs dérivés (Murk *et al*., Fundam. Appl. Toxicol., vol.33(1) (Sep. 1996), pp. 149-60 ; Bovee *et al*., Food. Addit. Contam., vol.15(8) (Nov-Dec.1998), pp. 863-75). Selon ce document, les inventeurs ont construit un plasmide d'expression dans lequel ils ont inséré les éléments génétiques de réponse à la dioxine en amont du gène reporteur de la luciférase. Le plasmide est transféré dans des cellules épithéliales de souris. Ce sont ces lignées cellulaires qui sont utilisées dans une méthode pour l'analyse quantitative de la teneur en hydrocarbone poly-aromatique dans un échantillon.

Un autre système connu sous le nom de "Tet-Lux" est basé sur un principe identique (Kurittu *et al*., J. Food Prot., vol.63(7) (Jul.2000), pp. 953-7). Dans cette application, c'est le système de régulation de la résistance à la tétracycline qui est cloné en amont d'un gène de luciférase en lieu et place du gène de résistance à la tétracycline. En présence de tétracycline dans le milieu de croissance des cellules recombinantes, le gène de luciférase peut s'exprimer et à l'aide de luciférine, de la lumière est émise. En absence de tétracycline, le système régulateur est fermé et aucune lumière n'est produite.

TetR-tetO constitue le système de régulation transcriptionnel le plus sensible connu à ce jour. La puissance des systèmes inductibles (Tet) à la tétracycline a également permis de développer une méthode de choix pour les recherches transgéniques. Ils sont fréquemment utilisés comme outil pour réguler des gènes cibles particuliers chez les eucaryotes (Stebbins *et al*., PNAS, vol.98(19) (2001) pp. 10775-80 ; Baron *et al*., Methods Enzymol. (2000) vol.327, pp. 401-421). Les auteurs rapportent qu'une faible présence de doxycycline ajoutée à l'alimentation normale de *drosophila* induit efficacement et rapidement les transgènes cibles chez les adultes, les larves et les embryons.

L'inconvénient majeur d'une méthode qui demande un développement cellulaire réside dans la durée nécessaire pour déclencher le signal mesurable. Il faut compter plusieurs multiplications cellulaires et attendre au moins une heure avant d'obtenir un signal interprétable. Dans certains cas l'échantillon peut aussi contenir des éléments susceptibles de modifier non spécifiquement la croissance des cellules recombinantes et de donner des erreurs ou d'ajouter du bruit à la mesure finale. De plus, ces cellules recombinantes sont parfois instables et peuvent être très sensibles à divers facteurs. Par ailleurs, leur utilisation dans une trousse d'essai n'est pas adaptée à une manipulation facile et pratique, surtout lorsqu'il faut faire fonctionner le test dans des conditions de terrain. De plus, ces types de test demandent souvent l'intervention d'un expert ou d'un opérateur qualifié pour réaliser l'analyse. Enfin, les réactifs et les instruments qui mesurent la lumière émise sont très onéreux.

### Buts de l'invention

La présente invention vise à fournir un nouveau procédé de diagnostic ne présentant pas les inconvénients et difficultés relevés dans l'état de la technique.

En particulier, l'invention vise à proposer l'utilisation totale ou partielle des éléments connus de régulation de l'expression des gènes pour le développement de procédés et/ou substances, utilisables *in vitro,* qui aurait comme champ d'application principalement la reconnaissance et la quantification de molécules.

Un but complémentaire de l'invention est de fournir un test rapide de diagnostic de molécules spécifiques.

Un but complémentaire de l'invention est de fournir un procédé et une trousse de diagnostic *in vitro* pour la détection et le dosage d'antibiotiques tels que les tétracyclines et les virginiamycines.

### Principaux éléments caractéristiques de l'invention

Un premier objet de la présente invention est de proposer une trousse de détection et/ou de quantification d'un ligand, se trouvant dans un échantillon d'analyse, par un récepteur. Le ligand est susceptible d'être présent dans un échantillon de matière biologique, qui est présenté la plupart du temps pour des raisons pratiques en solution. La trousse comprend les réactifs suivants :
- une séquence nucléotidique simple brin ou double brin, et
- ledit récepteur constitué d'une entité protéique monomérique ou multimérique comprenant au moins un premier site de reconnaissance spécifique pour ledit ligand et un deuxième site de reconnaissance spécifique pour ladite séquence nucléotidique.

La particularité du ligand est sa capacité de moduler positivement ou négativement la fixation du récepteur par son site de reconnaissance spécifique sur la séquence nucléotidique.

Avantageusement, le ligand est un antibiotique, appartenant de préférence à la famille des tétracyclines ou des virginiamycines, ou une substance apparentée. On choisit dès lors comme récepteur un répresseur de tétracycline (TetR) isolé parmi l'une quelconque des classes connues, c'est-à-dire A, B, C, D, E et G, ou de virginiamycine (VarR), qui est utilisé pour le dosage respectivement des tétracyclines et des virginiamycines.

Une caractéristique particulièrement avantageuse de l'invention est qu'elle comprend des moyens pour réaliser la quantification de l'antibiotique en un temps très court, de préférence moins de 15 minutes, et ce pour des concentrations avoisinant les limites maximales de résidus autorisées.

Le récepteur peut aussi être, selon l'invention, un récepteur nucléaire de la famille des régulateurs eucaryotiques de transcription, un récepteur de la famille des "récepteurs orphelins", leurs ligands étant aujourd'hui inconnus, ou encore un mutant des récepteurs naturels de tétracycline ou de virginiamycine, de récepteurs nucléaires ou de récepteurs orphelins.

Selon l'invention, la séquence nucléotidique est un fragment double brin de séquence spécifique des régions opératrices des gènes tetA et tetR pour la reconnaissance des tétracyclines ou varA et varR pour la reconnaissance des virginiamycines, ledit fragment étant de préférence positionné à l'extrémité 3' d'un élément constitué d'une biotine et d'une chaîne simple brin poly-T.

Selon une première forme d'exécution préférée de l'invention, la séquence nucléotidique est immobilisée, directement ou indirectement, sur un support solide, le récepteur étant éventuellement marqué par au moins une molécule.

Selon une deuxième forme d'exécution préférée de l'invention, le récepteur est immobilisé, directement ou indirectement, sur un support solide, de préférence à l'aide d'un anticorps spécifique et d'une protéine-A, la séquence nucléotidique étant éventuellement marquée par au moins une molécule.

Avantageusement, le récepteur ou la séquence nucléotidique, selon le cas, est marqué(e), directement ou indirectement, au moyen d'un anticorps ou une biotine. L'anticorps et la biotine peuvent éventuellement être marqués respectivement par l'intermédiaire d'une protéine-A et d'une molécule fixant la biotine. De préférence, ce marquage direct ou indirect est réalisé à l'aide de particules colorées telles que des particules d'or colloïdales, ou à l'aide d'une enzyme telle que la peroxydase.

Une caractéristique surprenante de l'invention est qu'aucun élément marqué n'est nécessaire pour une détection, la seule présence ou absence du complexe récepteur-fragment nucléotidique étant détectable par une méthode physico-chimique telle qu'une méthode du type Surface Plasmon Resonance ou la spectrométrie de masse.

De manière encore plus avantageuse, le fragment nucléotidique, couplé à une biotine, est associé à une protéine fixant le biotine, de préférence l'avidine, la streptavidine, la neutravidine ou un anticorps anti-biotine, pour former un complexe qui est déposé sur une membrane de nitrocellulose, définissant un premier point de capture pour le récepteur et la membrane de nitrocellulose comporte un second point de capture capable de récupérer, en tout ou en partie, l'excès de réactifs qui ne s'est pas fixé au premier point de capture.

De préférence, le second point de capture comprend de la gammaglobuline, de la protéine-A ou de l'antiprotéine-A.

De manière encore préférentielle, la trousse de l'invention comporte des moyens pour quantifier, après détection, les signaux obtenus aux deux points de capture soit par inspection visuelle, soit par mesure optique (réflectivité, absorbance, transmission, fluorescence, Digital Camera, chémoluminescence, etc.), soit par Surface Plasmon Resonance, soit encore par spectrométrie de masse.

Selon une caractéristique de l'invention, le récepteur comporte un troisième site de reconnaissance spécifique indépendant des sites de reconnaissance du récepteur pour la séquence nucléotidique et pour le ligand.

Le troisième site de reconnaissance spécifique du récepteur est avantageusement un site de reconnaissance pour une autre entité protéique, de préférence un anticorps ou un fragment spécifique d'anticorps, ou un site de fixation pour un ion métallique. L'autre entité protéique interagissant avec le troisième site de reconnaissance est marquée, de préférence au moyen de protéine-A associée à des particules d'or colloïdales.

L'invention peut être exploitée de manière extrêmement pratique lorsque le récepteur de la séquence nucléotidique et/ou l'anticorps et/ou un fragment de celui-ci est marqué par au moins une molécule permettant une détection par un procédé tel que l'immunoprécipitation, l'immunochromatographie, les détections de type ELISA ou RIA, la formation d'un précipité coloré résultant d'une réaction enzymatique, etc.

Toujours en considérant l'aspect pratique de l'invention, lesdits réactifs sont de préférence conditionnés de dans une fiole lyophilisée et sur un élément de type tigette immunochromatographique.

Alternativement, la trousse peut encore être dépourvue de fiole ; lesdits réactifs sont alors directement associés à l'intérieur de l'élément tigette sur une membrane proche de son extrémité qui peut entrer en contact avec un liquide d'analyse.

Un deuxième objet de la présente invention concerne un procédé de détection et/ou de quantification d'un ligand présent dans un échantillon de préférence biologique, mis en oeuvre au moyen de la trousse décrite ci-dessus et
caractérisé en ce que :
- l'on met en contact ledit échantillon avec la trousse et en ce que
- l'on détecte la présence ou l'on mesure la concentration en ligand présente dans ledit échantillon par immunochromatographie ou par dosage enzymatique, de préférence de type ELISA, par SPR ou par spectrométrie de masse.

Ce procédé présente l'avantage que l'échantillon biologique peut être directement mis en présence avec les réactifs de la trousse sans devoir être préalablement purifié. De plus, il n'y a pas de restriction quant à la nature de l'échantillon biologique utilisé, celui-ci pouvant comprendre la viande, le poisson, le sang, le sérum, un liquide physiologique, des urines, des larmes, de la salive, le lait, le miel, l'eau, des aliments pour bétail ou toute autre préparation alimentaire ou des liquides de culture et fermentation, ou encore des préparations ou dérivés de ces substances.

Enfin, bien que l'invention ait été décrite et illustrée dans ses formes d'exécution préférées et les plus accomplies, de nombreuses variantes paraissent possibles à l'évidence et s'inscrivent aussi bien dans le cadre' et l'esprit de la présente invention. En particulier, la dernière revendication couvre une large gamme d'équivalents relatifs à un procédé de détection et/ou de quantification d'une molécule ligand, mettant en oeuvre *in vitro,* entièrement ou en partie, une pluralité d'éléments, de préférence des procédés, mécanismes et/ou substances, connus comme appartenant à des mécanismes naturels de régulation de l'expression des gènes *in vivo.* Le procédé se caractérise de manière générique en ce qu'on utilise une entité protéique liante monomérique ou multimérique, appelée récepteur ou régulateur et originaire desdits mécanismes naturels de régulation génétique, comportant une pluralité de sites distincts de reconnaissance spécifique, l'état de réactivité de chaque dit site, vis-à-vis de son ligand propre, étant capable de moduler la réactivité d'au moins un autre site de reconnaissance dudit récepteur.

De préférence, ladite réactivité d'au moins un site de reconnaissance du récepteur protéique, vis-à-vis par exemple d'une séquence nucléotidique de type promoteur/opérateur génétique, peut être modulée par une modification de la distance centre à centre entre deux motifs HTH d'un dimère appartenant audit récepteur.

### Brève description des figures

La figure 1, dont il est fait mention précédemment, représente très schématiquement le mécanisme de régulation de la transcription génétique, tel qu'il se produit *in vivo.*

Les figures 2A et 2B, dont il est fait mention précédemment, représentent respectivement les mécanismes d'activation et d'inactivation du régulateur en présence de ligand.

La figure 3 représente la transposition schématique simplifiée des mécanismes illustrés dans les figures 2A et 2B selon la présente invention et ses diverses formes d'exécution préférées.

La figure 4 représente une courbe de dosage des tétracyclines dans le muscle de poulet, selon l'invention.

La figure 5 représente graphiquement un dosage de quatre molécules différentes de tétracycline par la méthode n° 2 de l'invention.

La figure 6 représente graphiquement les résultats obtenus par la technique de SPR (Surface Plasmon Résonance).

### Mécanismes génétiques à la base de l'invention

La figure 1 présente le schéma classique de régulation de la transcription d'un gène de structure 2. La transcription 5 de DNA en mRNA est initiée grâce à la fixation de la RNA polymérase 3 sur la région régulatrice en tête de gène 1 (promoteur/opérateur). La traduction 6 de mRNA 4 en protéines 7 est ensuite induite au niveau des ribosomes 4'.

L'activation du régulateur 8 en présence d'un ligand 9, inducteur, est décrite à la figure 2A. Lorsque le régulateur transcriptionnel inactif (RTI) 8 se fixe au promoteur 1, après avoir fixé le ligand 9, pour donner un régulateur transcriptionnel actif (RTA) 10, il y a activation ou non de la transcription du gène selon que RTA est respectivement un activateur ou un répresseur transcriptionnel.

L'inactivation du régulateur 8 en présence d'un ligand 9, co-répresseur, est décrite à la figure 2B. Lorsque le RTA 10 est fixé au promoteur 1, il y a activation de la transcription ou non selon que RTA est respectivement un activateur ou un répresseur transcriptionnel. La fixation du ligand 9 sur le RTA 10 provoque sa 'dissociation du promoteur 1, en RTI 8,9, ce qui induit respectivement la répression ou l'activation du gène.

### Description détaillée d'une forme d'exécution préférée de l'invention

La présente invention se base sur l'utilisation d'une entité protéique monomérique ou multimérique, généralement originaire des mécanismes connus de régulation génétique, qui porte plusieurs sites distincts de reconnaissance et dont l'état de réactivité d'un site, vis-à-vis de son propre ligand, peut moduler la réactivité d'un autre site de reconnaissance de cette même entité protéique. Dans le cas particulier de l'invention, il s'agit d'une reconnaissance pour un fragment d'acide nucléique d'une part et pour un ligand d'autre part, la fixation du ligand pouvant moduler négativement (c'est-à-dire empêcher) la reconnaissance de la séquence de DNA par l'entité protéique.

En d'autres termes, l'invention se base sur l'utilisation en technique de diagnostic *in vitro* de complexes du type "séquence de DNA - régulateur - ligand" pour la détection de substances apparentées à ou dérivant de la molécule de ligand.

La présente invention a l'avantage de ne pas avoir recours à l'expression d'un système biologique *in vivo,* et ne demande pas l'utilisation de lignées cellulaires particulières.

Pour autant, la présente invention exploite les propriétés connues des systèmes de régulation génétique mais dans une technologie totalement différente et beaucoup mieux adaptée à un test rapide. En effet les molécules biologiques sont mises en oeuvre *in vitro* sans avoir recours à aucune infrastructure ni développement cellulaire, ni expression de système biologique *in vivo.* Ce sont les trois éléments clés, à savoir acide nucléique/récepteur/ligand éventuel, originaires des systèmes de régulation, qui placés dans un environnement externe à toute viabilité cellulaire sont capables d'exprimer leur fonctionnalité en dehors du contexte cellulaire. Dans une application particulière, qui concerne le dosage des tétracyclines, les réactifs de base, présentés en un seul mélange, ont été directement repris dans l'échantillon d'analyse et la réponse a été obtenue en 5 minutes.

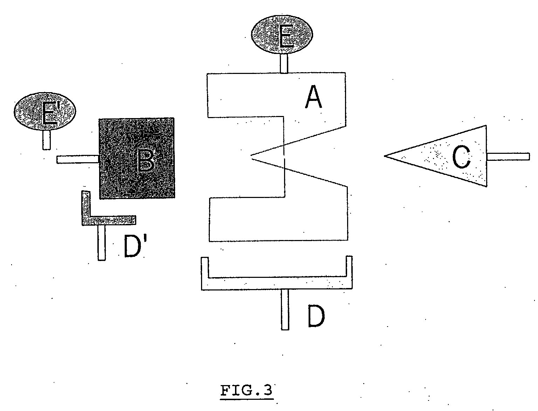

La figure 3 simplifie la description de l'invention en présentant les diverses approches proposées dans la présente demande de brevet. La figure 3 schématise dans sa plus simple expression, d'une part l'ensemble des éléments clés qui soutiennent l'invention et d'autre part l'ensemble des éléments indispensables pour détailler par la suite les formes d'exécution particulières de l'invention.

Les éléments clés sont au nombre de trois :
- premièrement une entité protéique capable de reconnaître une molécule liante et un fragment d'acide nucléique,
- deuxièmement une molécule liante (ligand) pouvant à son tour reconnaître l'entité protéique concernée et
- troisièmement un fragment d'acide nucléique, DNA ou mRNA, que l'entité protéique est également capable de reconnaître.

On a donc deux sites de reconnaissance distincts portés par une entité protéique qui peut se présenter sous la forme soit monomérique, soit multimérique, et de préférence multimérique.

Sur la figure 3, on distingue donc une entité protéique A capable de reconnaître spécifiquement une séquence d'acide nucléique B et de s'y fixer soit uniquement en la présence, soit uniquement en l'absence du ligand C. Lorsque C se fixe sur A, alors le complexe AC ne peut plus se fixer sur B ou encore A se détache de B. Dans le cas contraire (non illustré), c'est uniquement le complexe AC qui autorise A de se lier à B et en absence de C, A se désolidarise de B.

Un fragment B d'acide nucléique peut être ainsi reconnu par A, soit dans un premier cas uniquement en l'absence de C, soit dans un autre cas uniquement en la présence de C.

Un ligand C spécifique de A peut se fixer sur A quelque soit l'état de l'interaction de A et B. Sa fixation sur A empêche A de fixer B ou oblige le complexe AB préalablement formé de se dissocier, ou dans le cas contraire, c'est la formation du complexe AC qui autorise la fixation de AC sur B pour former le complexe ACB. Une diminution de la concentration de C oblige le complexe ACB à se dissocier complètement.

On peut également avoir un autre site D de reconnaissance, externe et indépendant du site de reconnaissance de A pour B et pour C. Ce peut être un site de reconnaissance pour une autre entité protéique, généralement un anticorps ou un fragment d'anticorps, capable de se fixer sur A indépendamment d'une fixation de B ou C sur A. Il peut également s'agir par exemple d'un site de fixation pour un ion métallique ou une autre protéine quelconque. De manière similaire, on peut avoir un autre site D' de reconnaissance du DNA (B) qui joue le même rôle que D mais vis-à-vis de B.

On peut encore avoir un lien artificiel E ajouté à A, soit par couplage chimique soit par exemple par ingénierie génétique ou protéique. Ce lien ne modifie pas la fonctionnalité de A (vis-à-vis de B, C et D) . Ce lien peut servir de point d'ancrage de A à une autre molécule quelconque soit pour marquer A ou pour fixer A à un support par exemple. Le lien artificiel E' joue le même rôle que E, mais vis-à-vis de B.

D'une manière générale, la présente invention s'applique aux techniques de diagnostic qui demandent habituellement deux éléments principaux, à savoir un élément de capture et un élément de marquage. Ces deux éléments suffisent pour décliner tout un ensemble de possibilités qui permettront *in fine* de décrire et de soutenir la thèse présentée dans la présente invention.

Selon la présente invention, tous les éléments décrits dans le schéma de la figure 3 (A, B, C, D, D', E et E'), sans aucune exception, peuvent ou ne peuvent pas, chacun, soit être immobilisés et servir de point de capture, soit être marqués. L'immobilisation directe ou indirecte sur un support quelconque permet la récupération éventuelle, partielle ou totale, du complexe en présence et ce, quel que soit l'état du complexe formé. Le marquage direct ou indirect par une quelconque molécule ou particule ou encore par un ensemble quelconque de molécules ou particules, permet d'identifier ou de visualiser directement ou indirectement la formation éventuelle du complexe en présence et ce, quel que soit l'état du complexe formé, sur l'élément de capture. Dans un cas particulier, l'élément de capture et/ou l'élément de marquage ne sont pas indispensables car c'est la simple présence ou absence physique du complexe formé qui est repéré par des techniques plus sophistiquées telles les techniques physico-chimiques de type spectrométrie de masse (SM) ou de type *Surface Plasmon Resonance* (SPR) par exemple.

En général, si un des éléments est immobilisé pour permettre la récupération éventuelle du complexe en présence, c'est un autre élément qui sera éventuellement modifié pour servir de marqueur du complexe en présence et vice versa.

Le marquage peut notamment être réalisé à l'aide de particules colorées pour une détection dans le cadre d'une application en immunochromatographie, à l'aide d'enzyme marqueur pour une détection dans le cadre d'une application de type ELISA, ou encore être réalisé par une molécule fluorescente pour une détection dans le cadre d'une application en détection de fluorescence. Dans le cas où des méthodes physico-chimiques seraient utilisées (par exemple : SM, SPR, etc.), le marquage externe n'est pas nécessaire car ces méthodes sont capables de repérer la présence ou l'absence de complexes moléculaires.

En général, l'immobilisation d'un quelconque des éléments peut s'effectuer sur un support :
- de type membrane naturelle ou synthétique (nylon, PVDF, nitrocellulose, etc.),
- de type surface plastique (polycarbonate, polystyrène, PVP, PVC, polypropylène, etc.),
- de type particules magnétiques, ou particules en latex, de type surface métallique ou en verre, en céramique ou en silice, etc. ,
   que ce soit directement ou indirectement par l'intermédiaire de chaînes de polymères (par ex. : dextran, PEO, ...) ou encore d'un "spacer" chimique quelconque.

### Exemples

La présente invention est illustrée par trois exemples clés dans lesquels on considère que le ligand du récepteur est la molécule à détecter dans l'échantillon d'analyse. En occurrence dans les exemples, le ligand est la tétracycline présente dans l'échantillon à analyser.

Le premier exemple illustre le dosage de la tétracycline dans la viande par une méthode immunochromatographique. Une variante de cet exemple fonctionne également pour le dosage dans le lait ou tout autre produit dérivé du lait, mais aussi dans le poisson, dans le sérum ou autre liquide physiologique, dans le sang, les urines, les larmes ou la salive. La technique décrite dans cet exemple peut également fonctionner en version ELISA ou le marquage du récepteur peut se faire par l'intermédiaire d'une enzyme (peroxydase ou phosphatase alcaline, etc.), soit directement, soit indirectement à l'aide du système biotine-avidine.

Le second exemple illustre le dosage de la tétracycline dans des produits laitiers par une méthode ELISA. Une variante de cet exemple fonctionne également pour le dosage dans la viande, le poisson, le sérum ou tout autre liquide physiologique, le sang, les larmes, les urines, la salive. La technique peut également fonctionner en version immunochromatographique où le marquage du DNA se fait alors par l'intermédiaire d'un anticorps anti-biotine ou d'une avidine ou streptavidine ou un de leurs dérivés, marqué à l'or colloïdal.

Le troisième exemple démontre qu'il est possible de détecter la présence de tétracycline en solution par l'intermédiaire d'une technique de type physico-chimique, de préférence la méthode de *Surface Plasmon Resonance* (SPR), sans devoir recourir à un marquage quelconque. En effet dans ce cas c'est la présence ou l'absence de complexe récepteur-DNA qui est directement mise en évidence.

### EXEMPLE 1

Cet exemple illustre la présente invention dans le cas du dosage *in vitro* de la tétracycline dans la viande (méthode immunochromatographique).

### Principe de base

Le fragment de DNA B est immobilisé et le récepteur A est marqué, soit directement par une biotine E, soit indirectement par un anticorps D et une protéine-A conjuguée à des particules d'or colloïdales. La tétracycline à détecter constitue le ligand C illustré dans la figure 3. Dans le présent exemple, l'outil technologique de révélation utilisé est un test immunochromatographique mais une version similaire par marquage enzymatique fonctionne également en ELISA. L'exemple illustre un dosage des tétracyclines en viande, mais le dosage fonctionne exactement de la même manière si l'échantillon est une autre matrice biologique quelconque.

On décrit ci-après la préparation des éléments nécessaires.

### 1.1 Le récepteur.

TetR est un récepteur à la tétracycline (Tc) particulièrement intéressant. C'est un homodimère constitué de 2 sous-unités identiques de 27 kD chacune. D'une part, il est spécifique à la tétracycline. En effet, ce récepteur protéique a comme fonction *in vivo* de fixer la tétracycline présente dans le cytoplasme ce qui déclenche le mécanisme de résistance à la tétracycline. D'autre part, il possède une affinité très élevée pour la tétracycline et ses dérivés (Ka valant de 10⁹ à 100 x 10⁹ M⁻¹). Le complexe formé présente également l'intérêt d'être relativement stable (t_{1/2} ≥ 2h à 37 °C). L'affinité de la Tc pour d'autres macromolécules comme les ribosomes, cibles cellulaires de ces antibiotiques, est nettement plus faible (10⁶ M⁻¹).

TetR est une protéine localisée dans le cytoplasme des bactéries Gram négatives résistantes à la tétracycline. C'est donc un homodimère (Hillen *et al*., J. Mol. Biol., 169 (1983), pp. 707 - 721) constitué de 2 sous-unités identiques, chacune possédant :
- une tête de fixation aux opérateurs des gènes tetA et tetR orientés en tandem, le motif hélice α - tournant β-hélice α (HTH) caractéristique des protéines se liant au DNA étant situé dans la région N-terminale de TetR ;.
- une poche de fixation au complexe (Mg-Tc)⁺ (Kaszycki *et al*., J. Prot. Chem., vol.15 (1996), pp. 607 - 619).

Seules les molécules de tétracycline complexées à un cation divalent (Mg²⁺) peuvent se fixer sur un dimère de TetR. Un dimère de TetR fixe 2 molécules de Tc avec la même affinité (Degenkolb *et al*., A.A.C., vol.35 (1991), pp. 1591-1595).

La formation du complexe TetR-Tc est optimale entre pH 7,5 et 12 et diminue à pH < 7,5. A pH 5, le répresseur est inactif (Hillen *et al*., J. Biol. Chem., vol.257 (1982), pp. 6605 - 6613). Le complexe reste stable jusqu'à 50°C.

Les complexes TetR-Tc et TetR-DNA ont été cristallisés et leurs structures tridimensionnelles sont connues à 2,5 Å de résolution (Hinrichs *et al*., 1994; Kisker *et al*., 1995, Orth *et al.,* 2000). On possède donc de nombreuses informations structurales sur ces complexes.

En absence de (Mg-Tc)⁺ dans TetR, les 2 domaines HTH peuvent se fixer au DNA avec une affinité très élevée estimée in vivo autour de 10¹¹ M⁻¹ (Orth *et al.,* 2000). Par contre en présence du complexe (Mg-Tc)⁺ dans TetR, la distance centre à centre entre les motifs HTH augmente de ∼5 Å, ce qui empêche le dimère TetR de se fixer sur la région opératrice du DNA. L'affinité de TetR pour le DNA est alors réduite d'un facteur ∼10⁹.

TetR-tetO constitue le système de régulation de la transcription inductible le plus efficace connu à ce jour, ce qui fait de TetR un récepteur de choix pour la tétracycline et de tetO une séquence nucléotidique d'ancrage potentiellement idéale pour TetR [Orth *et al*., 2000, Matthews *et al*., Nature Struct. Biol., vol.7(3) (Mar. 2000) pp.184-187].

Un système de régulation identique vient d'être décrit chez *Streptomyces,* comme mécanisme de résistance à la virginiamycine, un dimère VarR homologue à TetR et qui contient 2 motifs HTH capables de se fixer sur un opérateur en absence d'antibiotique. Par contre, quand la virginiamycine se lie sur VarR, un changement de conformation empêche ce dernier de se fixer sur le DNA (Namwat *et al*., J. Bac., vol.183 (Mar.2001), pp. 2025-2031).

### 1.1.1 Préparation du récepteur TetR

Dans l'exemple choisi, on a cloné le gène du récepteur de TetR de classe C contenu sur le plasmide pSC101 d*'E.coli* C600 (disponible chez American Type Culture Collection, Rockville, Maryland, USA) selon les méthodes bien connues par l'homme de l'art. Sur base de la séquence nucléotidique, deux amorces ont été sélectionnées et ont été utilisées pour l'amplification par PCR du gène qui a ensuite été cloné dans un plasmide d'expression pet12a.

Des cellules compétentes HB101 contenant le plasmide pT7pol23, codant pour la RNA polymérase du coliphage T7, sont transformées avec le produit de la ligation du fragment pcr obtenu et du pet12a. Les cellules mises en culture en milieu LB sont induites à DO₆₀₀ₙₘ = 0,8 par un changement de température à 42 °C pendant 3 heures.

La stratégie utilisée pour obtenir l'homogénéité protéique comporte 4 étapes qui sont respectivement :
I) une désintégration cellulaire à l'aide du système Constant Basic System,
II) un échangeur d'ions de type source 15Q PE 4.6/100 de 1,7 ml (Pharmacia),
III) un tamis moléculaire séphacryl S100 (Pharmacia) et
IV) une héparine HiTrap Heparin (Pharmacia).

En fin de purification, la protéine pure à 99% est alors stockée à une concentration de 21 µM en tampon phosphate de potassium 10 mM, pH 7,9, β-mercaptoéthanol 7 mM en présence de glycérol 50 % v/v et à -20 °C.

### 1.1.2 Marquage du récepteur

### 1.1.2.1 Marquage par biotinylation

Le récepteur reconnaissant la tétracycline, préparé ci-dessus, est modifié chimiquement en surface par ajout d'une biotine (vitamine H). Un dérivé N-hydroxysuccinimide ester (disponible chez Unisensor S.A.) a été utilisé. Dans un cas particulier, la biotinylation s'est opérée sur le dérivé cystéine par l'utilisation d'une biotine maléïmide disponible chez Pierce Inc., USA.

La biotinylation s'est effectuée selon la méthode décrite dans *Bioconjugate Techniques,* Hermanson, G.T. (1996), Academic Press, New York. Le récepteur biotinylé est alors dialysé contre son tampon de conservation constitué par du phosphate de potassium 20 mM, pH 7,9 et du β-mercaptoéthanol 14 mM. Après dialyse le récepteur est stocké dans ce même tampon dilué 2 fois en présence de glycérol 50 % v/v.

La mise en évidence de la biotine peut se faire par un anticorps antibiotine ou de la streptavidine ou de la neutravidine couplés à des particules colorées (or colloïdal, latex, cellulose) lorsqu'il s'agit d'une version de type immunochromatographique, ou couplés à une enzyme (HRP, PA, etc.) lorsqu'il s'agit d'une version de type ELISA.

### 1.2.2.2 Marquage à l'aide d'un anticorps anti-TetR

La préparation de l'anticorps s'est faite selon la méthode décrite dans Kachab, E.H. *et al.,* The Journal of Immunological Methods, vol.147, n°1 (Jan.1 1992), pp. 33-41.

La révélation des anticorps peut se faire soit après modification de ceux-ci par couplage direct à l'or (Method Mol. Biol. Totowa, N.J., vol.115 (1999), pp. 331-334 ; Colloidal Gold Principles, Methods and Applications, M.A. Hayat, Academic Press) ou par biotinylation et révélation par un anticorps antibiotine ou neutravidine couplés de manière enzymatique ou à l'aide de particules colorées, soit sans modifier l'anticorps en le révélant simplement par un anticorps anti-rabbit ou de la protéine-A également marquée enzymatiquement ou à l'aide de particules colorées.

Dans l'exemple précis, une partie de sérum non purifié obtenu ci-dessus est mise en présence d'une partie de récepteur TetR obtenu ci-dessus en 1.1.1, de 5 parties de protéine-A marquée à l'aide de particules d'or colloïdales de 40 nm et d'une DO à 500 nm de 10 (disponible chez Unisensor S.A., Liège, Belgium) et de 10 parties de tampon de lyophilisation (Tris 10 mM, pH 8, BSA 5 mg/ml, sucrose 10 g/l). Ce mélange est lyophilisé pendant 20 heures.

### 1.2 L'acide nucléique

Le dimère de TetR possède 2 motifs HTH capables de se fixer à une séquence spécifique de DNA, la région opératrice des gènes tetR et tetA.

Dans l'opéron de résistance à la Tc du transposon Tn10, les régions opératrices des gènes tetA et tetR qui fixent le récepteur TetR sont localisées et leurs séquences connues (Hillen *et al.*, 1984).

Par alignement de séquence, on a également localisé cette séquence conservée dans l'opérateur tetA du pSC101 et fait synthétiser un oligonucléotide de 31 pb englobant cette région consensus (teta-40) ainsi qu'un autre oligonucléotide de 31 pb de séquence complémentaire au premier (teta-30). Le premier oligonucléotide (teta-40) possédera en plus une queue poly-T en 5' (10T), qui restera simple brin lors de l'appariement des deux oligonucléotides, ainsi qu'une biotine à cette extrémité 5'.

Un oligonucléotide synthétique de 31 pb possédant un site de fixation de TetR sur l'opérateur tetA du pSC101 ainsi que son complémentaire biotinylé en 5' ont été synthétisés (Eurogentec, Belgium) :

Teta 40 : 5'biotine-TTTTTTTTTTAATGCGGTAGTTTATCACAGTTAATTGCTAA 3'

Teta 30 : 3′ TTACGCCATCAAATAGTGTCAATTAACGATT 5′

### 1.2.1 Préparation de l'acide nucléique

Pour l'hybridation, les deux oligonucléotides complémentaires sont mélangés en quantité équimolaire (2 x 10⁻⁴ M) en NaCl 0,5 M. Le mélange est incubé 5 minutes dans l'eau bouillante puis refroidi lentement dans ce bain marie jusqu'à température ambiante afin de réduire les appariements non spécifiques.

### 1.2.2 Immobilisation du fragment d'acide nucléique

Le fragment d'acide nucléique est immobilisé sur l'avidine par l'extrémité d'un des brins qui contient une biotine. L'avidine est une glycoprotéine tétramérique isolée du blanc d'oeuf. Chaque sous-unité a une masse moléculaire de 16,4 kD et fixe une molécule de biotine de manière non covalente avec une affinité particulièrement élevée (Ka = 10¹⁵ M⁻¹).

Les deux oligonucléotides teta30 et teta40-biotine, préalablement hybridés à 10⁻⁴ M en NaCl 0,5 M, sont mis en présence d'avidine à 20 mg/ml en eau dans un rapport 10/1 v/v. Le produit de la réaction est déposé sur une membrane de nitrocellulose à l'aide d'un système de dépose commercialisé par BioDot Inc., USA.

### 1.3 Description de la technique utilisée

La technique immunochromatographique est connue et décrite dans la littérature (Developing Immunochromatographic Test Strips : A short Guide, Millipore, Lit. No. TB500 Printed in USA 11/96, 96-204). Dans le cas précis de l'a présente invention, le mélange obtenu ci-dessus (voir point 1.2.2) est déposé sur une membrane en nitrocellulose. Il est connu que les protéines, et ici notamment l'avidine, se fixent par interaction dipôle de manière irréversible sur le support en nitrocellulose. Par ailleurs, il est connu que les acides nucléiques sous forme de doubles brins n'entrent pas en interaction avec ce même support. En d'autre termes, le mélange de l'avidine - biotine - acide nucléique est un choix favorable où l'avidine sert d'ancrage et le DNA reste libre et accessible pour la reconnaissance par une protéine, en l'occurrence dans le présent exemple, le récepteur TetR.

La membrane en nitrocellulose est ensuite placée en contact aux deux extrémités par un papier absorbant quelconque. Sur la nitrocellulose se trouvent deux points de capture, l'un est constitué du mélange avidine - biotine-DNA décrit au point précédent et l'autre, placé après le premier en se référant au sens de migration du liquide, est une protéine capable d'être reconnue par de la protéine-A. De préférence cette protéine est une gammaglobuline. Ceci constitue l'élément tigette utilisé dans l'Exemple 1.

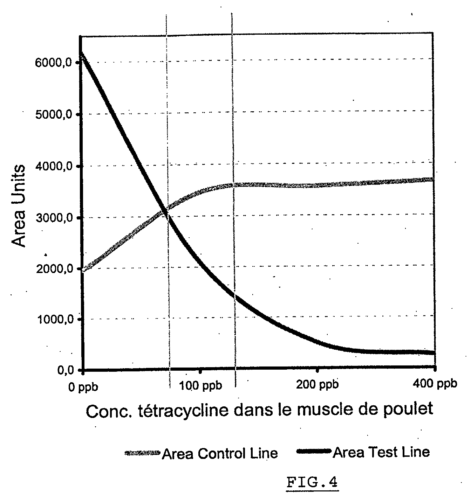

Sur cet élément tigette, le premier point de capture constitue la zone "test" (Test Line) spécifique de la présence ou de l'absence de tétracycline dans le milieu réactionnel et le second point de capture est la zone de "référence" (Control Line) capable de récupérer l'excès de réactif non retenu au premier point de capture. L'appréciation de la quantité de tétracycline dans l'échantillon d'analyse se fait par interprétation optique de la surface des traits présents aux points de capture (voir figure 4).

### 1.4 Dosage des tétracyclines en viande par la méthode 1

A 25 g de muscle de poulet sont ajoutés 75 ml de tampon MacIlvain pH 4 (1 litre d'une solution d'acide citrique monohydrate à une concentration de 21 g/l et 625 ml d'une solution de NaHPO4 anhydre à une concentration de 28,4 g/l, le pH étant ajusté à la valeur de 4 à l'aide de NaOH 0,1M) dilué quatre fois dans l'eau. Le mélange est haché à l'aide d'un hachoir ménager de type SEB Rondo 500 pendant 2 minutes. Le broyat est repris dans un Eppendorf pour être centrifugé 2 minutes à 6000 tpm dans une centrifugeuse de type Eppendorf. 200 µl de surnageant sont ensuite incubés en présence du lyophilisat préparé ci-dessus (voir point 1.1.2.2). Après deux minutes d'incubation à température ambiante (± 20°C), la tigette d'analyse décrite ci-dessus (en 1.3) est plongée dans la solution. L'interprétation finale est faite après 8 minutes d'incubation à l'aide d'un lecteur optique de tigette disponible chez 77-Elektronika (Budapest, Hungary). Les résultats sont repris à la figure 4. Avec cette méthode, des échantillons de poulet contenant une faible quantité de tétracycline de l'ordre de 100 ppb peuvent être facilement détectés en moins de 15 minutes. La présente invention concerne le dosage des tétracyclines à une valeur légèrement inférieure à la Limite Maximale de Résidu (LMR) autorisée en Europe. En dessous de cette valeur le test donne un signal négatif et au dessus de cette valeur le test donne un résultat positif.

Des résultats similaires ont été obtenus avec du muscle, du rein et du foie en provenance de porc, de poulet, de boeuf mais également avec des échantillons de poisson et des oeufs. Dans le cas où l'on souhaite respecter la LMR d'application pour le rein, le foie et les oeufs, il y a lieu de diluer l'échantillon respectivement six (6) fois, trois (3) fois et deux (2) fois en tampon MacIlvain décrit ci-dessus, dilué huit fois dans l'eau. Pour les oeufs il n'est pas nécessaire de broyer.

Il est remarquable de constater que la présente invention propose, à partir d'un échantillon "solide", en l'occurrence un morceau de viande, une méthode de quantification d'antibiotique qui peut être réalisée en un temps très court (moins de 15 minutes) et ce pour des concentrations avoisinant les ou inférieures aux limites maximales de résidus (LMR) autorisées.

### 1.5. Dosage des tétracyclines en lait par la méthode 1

200 µl de lait sont incubés en présence des réactifs lyophilisés et préparés comme au point 1.1.2.2. Après deux minutes d'incubation à 37 °C, la tigette d'analyse décrite en 1.3 est plongée dans la solution. L'interprétation finale est faite après 8 minutes d'incubation à l'aide d'un lecteur optique de tigette disponible chez 77-Elektronika (Budapest, Hungary). Les résultats sont repris dans le tableau I. A 50 ppb, le signal "test" est inférieur au signal "référence" et le test donne un résultat positif.

**Tableau I**

| Concentration tétracycline en lait (ppb) | Intensité zone Test | Intensité zone Référence |
|---|---|---|
| 0 | 5218 | 1837 |
| 10 | 4357 | 1373 |
| 20 | 3620 | 1787 |
| 30 | 3041 | 1921 |
| 40 | 2273 | 2080 |
| 50 | 2023 | 2402 |
| 60 | 1096 | 2414 |
| 70 | 985 | 2189 |
| 80 | 630 | 2367 |
| 90 | 377 | 2190 |
| 100 | 399 | 2123 |

### EXEMPLE 2

L'exemple 2 illustre la présente invention pour le dosage des tétracyclines dans le lait (méthode ELISA).

### Principe de base

Dans cet exemple, en reprenant la nomenclature de la figure 3, le récepteur A est immobilisé à l'aide d'un anticorps D et d'une protéine-A et le DNA B est marqué à l'aide d'une biotine E' et d'avidine peroxydase. Dans le présent exemple, l'outil technologique de révélation utilisé est un test ELISA mais dans le cas où le marquage du DNA serait fait à l'aide de particules colorées, des résultats similaires peuvent être obtenus en immunochromatographie. Dans l'exemple 2, des tubes ou des plaques ELISA sont utilisés. L'exemple 2 illustre un dosage des tétracyclines dans le lait, mais le dosage fonctionne exactement de la même manière si l'échantillon est une autre matrice biologique quelconque.

On décrit ci-après la préparation des éléments nécessaires.

### 2.1 Le récepteur

Le récepteur TetR est produit et purifié selon la méthode décrite au point 1.1.1.

### 2.1.1 Préparation du support ELISA pour la récupération du récepteur

1µg de protéine-A présent dans 100 µl de tampon de "coating" (tampon carbonate 0,05 M pH 9,6) est déposé dans chaque puits d'une microplaque disponible chez Nunc, Maxisorp F8 (ref. : 468667A) et incubé une nuit à 4 °C. Le lendemain le surnageant est aspiré.

Les puits sont saturés pendant 30 min à température ambiante avec 200 µl de tampon phosphate 0,01 M pH 7,4 BSA 0,5 %, après quoi le surnageant est aspiré et les puits sont lavés avec du NaCl 0,15 M Tween 0,5 %.

Enfin, 200 µl de tampon phosphate 0,01 M pH 7,4 saccharose 1% sont ajoutés et incubés 30 min à température ambiante. Les puits sont ensuite lavés et séchés à l'air comprimé et conservés dans un dessicateur à 4 °C à l'abri de l'humidité.

### 2.2 Préparation du DNA marqué à la peroxydase.

Il s'agit de la même préparation décrite dans l'exemple précédent au point 1.2.1 qui est mise en présence d'avidine peroxydase disponible chez Pierce Inc. (Immunopure Avidin-Horseradish Peroxydase Conjugated ref. n° 21123). Le mélange réactionnel décrit ci-après met en présence 10 picomoles de DNAds-biotine pour 0,61 picomoles d'avidine-peroxydase.

### 2.3 Préparation du mélange réactionnel

Le mélange réactionnel est préparé de la manière suivante : 1 ml de solution contient :
I) 4,76 µl de récepteur TetR 21 µM préparé comme ci-dessus (en tampon phosphate 10 mM, pH 7,9, β-mercaptoéthanol 7 mM, Glycérol 50 % v/v) ;
II) 2 µl d'une solution de sérum se composant de : 100 µl de sérum préparé comme en 1.1.2.2, 900 µl de tampon phosphate 10 mM, pH 7 et 1 ml de glycérol ;
III) 1 µl de DNA biotinylé comme décrit en 1.2 (10⁻⁴ M en NaCl 0,5 M) ;
IV) 2 µl d'une solution d'avidine HRP se composant de 100 µl d'avidine HRP 1 mg/ml, 900 µl de tampon phosphate 10 mM, pH 7 et de 1 ml de glycérol ;
V) 990,24 µl de tampon phosphate 50 mM, pH 7,4, BSA 0.5 %, MgCl₂ 10 mM.

### 2.4 Dosage des tétracyclines dans le lait par la méthode 2

50 µl de lait à analyser sont mis en présence de 100 µl du mélange réactionnel décrit ci-dessus dans un puits préalablement "coaté" à la protéine-A (2.1.1). Le mélange est incubé 10 min à température ambiante sur une table agitante. Le puits est ensuite lavé 4 fois avec la solution de lavage (NaCl 0,15 M Tween 0,5 %). Ensuite 150 µl de tétraméthylbenzydine/H₂O₂ 50/50 (KPL Inc., USA) sont ajoutés dans le puits et l'incubation se prolonge pendant 30 min à l'obscurité. La réaction de coloration est arrêtée avec 50 µl de H₂SO₄ 6 M et la lecture est faite à l'aide d'un spectrophotomètre à 450 nm.

### 2.5 Dosage des tétracyclines en viande par la méthode 2

Une variante de cet exemple 2 montre le dosage des tétracyclines en viande par une méthode ELISA. Dans ce cas, l'échantillon est préparé comme suit : 5 g de viande ajoutés à 15 ml de tampon MacIlvain pH 4 (tel que décrit au point 1.4) sont broyés à l'ultra-turrax à 2000 tpm pendant 30 secondes. 2 g (∼2 ml) de broyat correspondant à 0,5 g de viande sont centrifugés 30 min à 13000 tpm (15000 g) à 4 °C. Le surnageant, contenant l'extraction des tétracyclines contenues dans 0,5 g de viande est déposé sur une colonne C18 Bond Elut (Varian) 100 mg préalablement conditionnée avec 1 ml de méthanol suivi de 1 ml de tampon MacIlvain. Après lavage de la colonne par 1 ml H₂O et séchage de la colonne par centrifugation 5 min à 4000 g (4 °C) , l'élution est réalisée par 1 ml de méthanol. Après évaporation du méthanol, le résidu est repris dans 50 µl de tampon phosphate 0,01 M, pH 7,4, BSA 0,5 %. A ce stade, l'échantillon est prêt pour le dosage selon la méthode décrite ci-dessus dans laquelle les 50 µl d'échantillon extrait remplacent les 50 µl de lait.

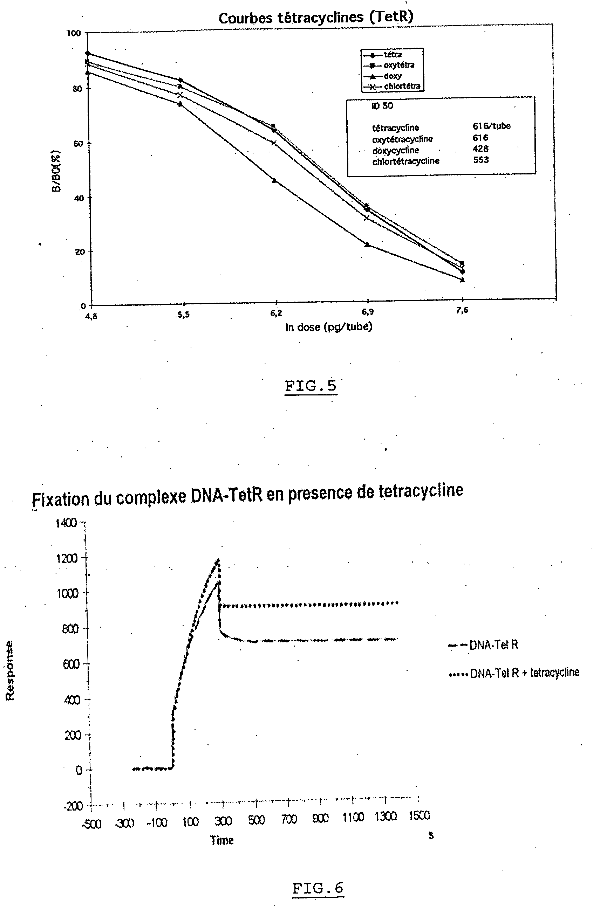

La figure 5 illustre à titre exemplatif le dosage de quatre molécules de tétracycline différentes par la même méthode. B est l'absorbance obtenue en l'absence d'antibiotique et B est l'absorbance obtenue en présence d'antibiotique. L'ID 50 correspond à la dose d'antibiotique nécessaire pour obtenir 50 % de saturation du récepteur.

### EXEMPLE 3

L'exemple 3 illustre la présente invention pour le dosage des tétracyclines sans devoir recourir ni à un marquage par particules colorées, ni à un marquage enzymatique. C'est uniquement la présence du complexe formé qui est détectée. Dans cet exemple, la reconnaissance du complexe formé est faite de manière indirecte à l'aide de la technique *Surface Plasmon Resonance* (SPR technology, Biacore A.B., Uppsala, Sweden).

### Principe de base

Dans cet exemple, en reprenant la nomenclature de la figure 3, le fragment de DNA B (préparé en 1.2.1) s'immobilise sur le chip Biacore Streptavidine et le récepteur A (préparé en 1.1.1) est utilisé pur sans avoir été marqué. En absence de tétracycline dans le milieu, le complexe récepteur-DNA se forme, ce qui empêche le DNA complexé au récepteur de se fixer sur le chip. En présence de tétracycline, le DNA libre a toujours la liberté de se fixer sur la streptavidine et c'est la fixation du DNA sur le chip qui génère l'augmentation du signal.

### Mode opératoire et résultat

Le chip est conditionné dans le tampon de réaction qui est constitué d'une solution d'HEPES 75 mM, pH 7,5, NaCl 65 mM, MgCl₂ 15 mM et BSA 1 mg/ml.

Une première solution notée "FC1" de 323,1 µl est constituée de 1,9 µl de DNA préparé comme en 1.2.1, 10 µl de TetR 2,1 µM préparé comme en 1.1.1 et de 311 µl de tampon de réaction.

Une seconde solution notée "FC2" de 323,1µl est constituée de 1,9 µl de DNA, 10 µl de TetR 2,1 µM, de 4,2 µl de tétracycline 10 µM et de 307 µl de tampon de réaction.

Les solutions précitées sont incubées 5 min à température ambiante avant d'être injectées dans les deux canaux respectifs pendant 5 minutes à 10 µl/min. Dans cette expérience, on montre à la figure 6 qu'une concentration équivalente à 60 ppb de tétracycline, présente dans la solution d'analyse, n'autorise pas la formation du complexe récepteur-DNA, rendant libre le DNA qui est capable de se fixer à la streptavidine immobilisée sur le chip. Dans le cas FC1, c'est-à-dire en absence de tétracycline, le récepteur forme un complexe avec le DNA, empêchant celui-ci de se fixer à la streptavidine immobilisée sur le chip. Ces résultats sont un peu inattendus car c'est différent de ce qui est observé dans les exemples 1 et 2.

## Revendications

1. Trousse de détection et/ou de quantification d'un ligand (C), présent dans un échantillon d'analyse, par un récepteur (A), **caractérisée en ce qu'**elle comprend les réactifs suivants :
- une séquence nucléotidique (B) simple brin ou double brin, et
- ledit récepteur (A) constitué d'une entité protéique monomérique ou multimérique comprenant au moins un premier site de reconnaissance spécifique pour ledit ligand (C) et un deuxième site de reconnaissance spécifique pour ladite séquence nucléotidique (B),
ledit ligand (C) étant capable de moduler positivement ou négativement la fixation du récepteur (A) par son site de reconnaissance spécifique sur la séquence nucléotidique (B).

2. Trousse selon la revendication 1, **caractérisée en ce que** le ligand (C) est un antibiotique, appartenant de préférence à la famille des tétracyclines ou des virginiamycines, ou une substance apparentée.

3. Trousse selon la revendication 1 ou 2, **caractérisée en ce que** le récepteur (A) est un répresseur de tétracycline (TetR) isolé parmi l'une quelconque des classes connues ou de virginiamycine (VarR) et est utilisé pour le dosage respectivement des tétracyclines et des virginiamycines.

4. Trousse selon l'une quelconque des revendications 1 à 3, **caractérisée en ce qu'**elle comprend des moyens pour réaliser la quantification de l'antibiotique en un temps très court, de préférence moins de 15 minutes et ce pour des concentrations avoisinant les limites maximales de résidus (LMR) autorisées.

5. Trousse selon la revendication 1 ou 2, **caractérisée en ce que** le récepteur (A) est un récepteur nucléaire de la famille des régulateurs eucaryotiques de transcription.

6. Trousse selon la revendication 1 ou 2, **caractérisée en ce que** le récepteur (A) est un récepteur de la famille des "récepteurs orphelins", leurs ligands étant aujourd'hui inconnus.

7. Trousse selon l'une quelconque des revendications 1 à 6, **caractérisée en ce que** le récepteur (A) est un mutant des récepteurs naturels de tétracycline ou de virginiamycine, des récepteurs nucléaires ou des récepteurs orphelins.

8. Trousse selon l'une quelconque des revendications 1 à 7, **caractérisé en ce que** la séquence nucléotidique (B) est un fragment double brin de séquence spécifique des régions opératrices des gènes tetA et tetR pour la reconnaissance des tétracyclines ou varA et varR pour la reconnaissance des virginiamycines, ledit fragment étant de préférence positionné à l'extrémité 3' d'un élément constitué d'une biotine et d'une chaîne simple brin poly-T.

9. Trousse selon l'une quelconque des revendications 1 à 8, **caractérisée en ce que** la séquence nucléotidique (B) est immobilisée, directement ou indirectement, sur un support solide, le récepteur (A) étant éventuellement marqué par au moins une molécule (E,D).

10. Trousse selon l'une quelconque des revendications 1 à 8, **caractérisée en ce que** le récepteur (A) est immobilisé, directement ou indirectement, sur un support solide, de préférence à l'aide d'un anticorps spécifique et d'une protéine-A, la séquence nucléotidique (B) étant éventuellement marquée par au moins une molécule (E',D').

11. Trousse selon la revendication 9, **caractérisée en ce que** le récepteur (A) est marqué, directement ou indirectement, au moyen d'un anticorps (D) ou une biotine (E), ledit anticorps (D), respectivement ladite biotine (E), pouvant à son tour être marqué(e) par l'intermédiaire d'une protéine-A, respectivement par une molécule fixant la biotine, ledit marquage du récepteur (A), de l'anticorps (D) ou de la biotine (E) étant réalisé à l'aide de particules colorées, de préférence des particules d'or colloïdales, ou à l'aide d'une enzyme, de préférence la peroxydase.

12. Trousse selon la revendication 10, **caractérisée en ce que** la séquence nucléotidique (B) est marquée, directement ou indirectement, au moyen d'un anticorps (D') ou une biotine (E'), ledit anticorps (D'), respectivement ladite biotine (E') pouvant à son tour être marqué(e) par l'intermédiaire d'une protéine-A, respectivement par une molécule fixant la biotine, ledit marquage de la séquence nucléotidique (B), de l'anticorps (D') ou de la biotine (E') étant réalisé à l'aide de particules colorées, de préférence des particules d'or colloïdales, ou à l'aide d'une enzyme, de préférence la peroxydase.

13. Trousse selon la revendication 9 ou 10, **caractérisé en ce qu'**aucun élément marqué n'est nécessaire pour une détection, la présence ou l'absence seule du complexe récepteur (A) - fragment nucléotidique (B) étant détectable par une méthode physico-chimique, de préférence une méthode de Surface Plasmon Resonance (SPR) ou de spectrométrie de masse.

14. Trousse selon la revendication 9, **caractérisée en ce que** le fragment nucléotidique (B), couplé à une biotine (E), est associé à une protéine fixant la biotine, de préférence l'avidine, la streptavidine, la neutravidine ou un anticorps anti-biotine, pour former un complexe qui est déposé sur une membrane de nitrocellulose, définissant un premier point de capture pour le récepteur (A).

15. Trousse selon la revendication- 14, **caractérisée en ce que** la membrane de nitrocellulose comporte un second point de capture capable de récupérer, en tout ou en partie, l'excès de réactifs qui ne s'est pas fixé au premier point de capture, ledit second point de capture comprenant de préférence de la gammaglobuline, de la protéine-A ou de l'anti-protéine-A.

16. Trousse selon la revendication 15, **caractérisée en ce que**, après détection, elle comporte des moyens pour quantifier les signaux obtenus aux deux dits points de capture, de préférence par interprétation visuelle, par mesure optique, et encore de préférence par réflectivité, absorbance, transmission, fluorescence, Digital Camera, chémoluminescence, par Surface Plasmon Resonance ou par spectrométrie de masse.

17. Trousse selon l'une quelconque des revendications précédentes, **caractérisée en ce que** le récepteur (A) comporte un troisième site de reconnaissance spécifique indépendant des sites de reconnaissance du récepteur (A) pour la séquence nucléotidique (B) et pour le ligand (C).

18. Trousse selon la revendication 17, **caractérisée en ce que** le troisième site de reconnaissance spécifique du récepteur (A) est un site de reconnaissance pour une autre entité protéique (D), de préférence un anticorps ou un fragment spécifique d'anticorps, ou un site de fixation pour un ion métallique (D).

19. Trousse selon la revendication 18, **caractérisée en ce que** ladite autre entité protéique (D) interagissant avec le troisième site de reconnaissance est marquée, de préférence au moyen de protéine-A associée à des particules d'or colloïdales.

20. Trousse selon la revendication 18 ou 19, **caractérisée en ce que** le récepteur (A) de la séquence nucléotidique (B) et/ou l'anticorps et/ou un fragment de celui-ci (D) est marqué par au moins une molécule permettant une détection par au moins un procédé choisis dans le groupe constitué de l'immunoprécipitation l'immunochromatographie, les détections de type ELISA ou RIA et la formation d'un précipité coloré résultant d'une réaction enzymatique.

21. Trousse selon la revendication 1, **caractérisée en ce que** lesdits réactifs sont conditionnés dans une fiole lyophilisée et sur un élément de type tigette immunochromatographique.

22. Trousse selon la revendication 21, **caractérisée en ce qu'**elle est dépourvue de fiole et **en ce que** lesdits réactifs sont directement associés à l'intérieur dudit élément tigette sur une membrane proche de son extrémité qui peut entrer en contact avec un liquide d'analyse.

23. Trousse selon la revendication 20 ou 21, **caractérisée en ce que** ledit élément de type tigette immunochromatographique est formé d'un support linéaire comprenant une membrane de nitrocellulose couplée à un papier absorbant présentant deux points de capture, un premier point de capture pour récupérer en tout ou en partie le complexe séquence nucléotidique (B) - récepteur (A) - ligand (C) et un second point de capture pour récupérer tout excès de réactifs ne s'étant pas fixé au premier point de capture.

24. Procédé de détection et/ou de quantification d'un ligand (C) présent dans un échantillon, de préférence biologique, au moyen de la trousse selon l'une quelconque des revendications 1 à 23, **caractérisé en ce que** :
- l'on met en contact ledit échantillon avec la trousse et **en ce que**
- l'on détecte la présence ou l'on mesure la concentration en ligand (C) présente dans ledit échantillon par immunochromatographie, par dosage enzymatique, de préférence de type ELISA, par SPR ou par spectrométrie de masse.

25. Procédé selon la revendication 24 **caractérisé en ce que** l'échantillon biologique est directement mis en présence avec les réactifs sans devoir être préalablement purifié.

26. Procédé selon la revendication 24 ou 25, **caractérisé en ce que** l'échantillon biologique comprend de la viande, du poisson, du sang, du sérum, du liquide physiologique, des urines, des larmes, de la salive, du lait, du miel, de l'eau, des aliments pour bétail ou d'autres préparations alimentaires ou des liquides de culture et de fermentation, ou encore des préparations ou dérivés de ces substances.

27. Procédé de détection et/ou de quantification d'une molécule ligand (C), mettant en oeuvre *in vitro,* entièrement ou en partie, une pluralité d'éléments, de préférence des procédés et/ou des substances, connus comme appartenant à des mécanismes naturels de régulation de l'expression des gènes *in vivo,* **caractérisé en ce qu'**on utilise une entité protéique liante monomérique ou multimérique, appelée récepteur ou régulateur (A) et originaire desdits mécanismes naturels de régulation génétique, comportant une pluralité de sites distincts de reconnaissance spécifique, l'état de réactivité de chaque dit site, vis-à-vis de son ligand propre, étant capable de moduler la réactivité d'au moins un autre site de reconnaissance dudit récepteur (A).

28. Procédé selon la revendication 27, **caractérisé en ce que** ladite réactivité d'au moins un site de reconnaissance du récepteur (A) est modulée par une modification de la distance centre à centre entre deux motifs HTH d'un dimère appartenant audit récepteur (A).
